# EUROPEAN PATENT SPECIFICATION

(11) **EP 2 698 145 B1**
(45) Date of publication and mention of the grant of the patent: **02.12.2020**
(21) Application number: 13180676.2
(22) Date of filing: 16.08.2013
(51) Int. Cl.: A61P 25/24

(54) **Pharmaceutical Formulations comprising dapoxetine and a PDE5 inhibitor**
Pharmazeutische Formulierungen enthaltend Dapoxetin und einen PDE5 Inhibitor
Formulations pharmaceutiques comprenant de la dapoxétine et un inhibiteur de la PDE-5.

(30) Priority: 17.08.2012 TR 201209599
(43) Date of publication of application: 19.02.2014
(73) Proprietor: MONTERO GIDA SANAYI VE TICARET A.S., Istinye, Istanbul 34460 (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Mutlu, Aydin

(56) References cited:
- EP-A1- 2 316 435
- WO-A1-2007/002125
- DE-A1-102009 058 321
- US-A1- 2002 037 828
- LU SHAOMING ET AL: "Combined use of phosphodiesterase-5 inhibitors and selective serotonin reuptake inhibitors for temporary ejaculation failure in couple undergoing assisted reproductive technologies", FERTILITY AND STERILITY, ELSEVIER SCIENCE INC, NEW YORK, NY, USA, vol. 91, no. 5, 1 May 2009 (2009-05-01), pages 1806-1808, XP002570491, ISSN: 0015-0282

## Description

### Technical field of the invention

The present invention is related to formulations containing a combination of dapoxetine or a pharmaceutically acceptable salt thereof with a phosphodiesterase Type 5 inhibitor or a pharmaceutically acceptable salt thereof. The invention is also related to a process for preparation a similar formulation and its use in treatment of erectile dysfunction.

### The background of the invention

Selective serotonin reuptake inhibitors (SSRI) are used in many types of depression including the endogenous type, long-term prophylaxis in recurrent depressions, obsessive-compulsive disorders, panic attacks, social fobias, and blumia nervosa. Dapoxetine, first described at European patent application no EP 0288188 B1, is a selective serotonin reuptake inhibitor. Dapoxetine is used for treatment of depression and premature ejaculation and its chemical formula is shown in formula I. In addition, it was approved in Sweden and Finland for the treatment of premature ejaculation.

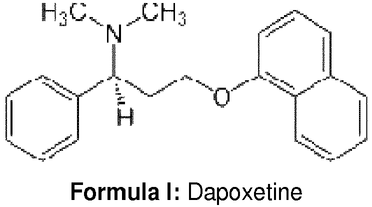

Dapoxetine is rapidly absorbed following oral administration and undergoes a rapid disintegration by near-complete binding to plasma proteins. Therefore, it reaches its peak plasma concentration (Cmax) 1 hour following oral administration. Orally given tablets are available in the market with the commercial name Priligy® containing 30 mg or 60 mg dapoxetine hydrochloride as active substance and lactose monohydrate, microcrystalline cellulose, croscarmellose sodium, colloidal anhydrase cilica, magnesium stearate, hypromellose, titanium dioxide (E171), triacetine, black iron oxide (E172), and yellow iron oxide as inactive ingredients.

The most common problem with oral formulations of dapoxetine is its bitter taste. To mask its bitter taste, the tablets are generally coated with coating agents. In addition, sweetener mixtures or cahion exchange resins have been used.

Phosphodiesterase type 5 inhibitors (PDE5 inhibitor) are used in treatment of erectile dysfunction (ED). PDE5 inhibitors selectively and effectively block phosphodiesterase enzyme and increase cyclic guanosine monophosphate (cGMP) levels in cavernous smooth muscle cells. The most commonly used phosphodiesterase inhibitors are avanafil, lodenafil, mirodenafil, sildenafil, tadalafil, vardenafil, and udenafil.

Avanafil is a PDE5 inhibitor used in the ED treatment and sold under the brand name of Stendra®. It exerts a more rapid effect compared to other PDE5 inhibitors. Its chemical name is (S)-4-[(3-Chloro-4-methoxybenzyl)amino]-2-[2-(hydroxymethyl)-1-pyrrolidinyl]-N-(2pyrimidinylmethyl)-5-pyrimidinecarboxiamide. Its chemical Formula is shown in Formula II.

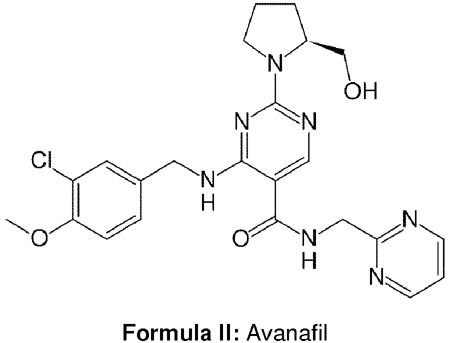

Lodenafil is a PDE5 inhibitor used in the ED treatment sold under the brand name of Helleva®. It has been formulated as a dimer and it is transformed into two active lodenafil molecules after it is taken into the body. This increases its bioavailability. Its chemical name is bis-(2-{4-[4-ethoxy-3-(1-methyl-7-oxo-3-propyl-6,7-dihydro-1H-pyrazole[4,3-d]pyrimidine-5-il)-benzenesulfonyl]piperazine-1-il}-ethyl)carbonate. Its chemical structure is given in Formula III.

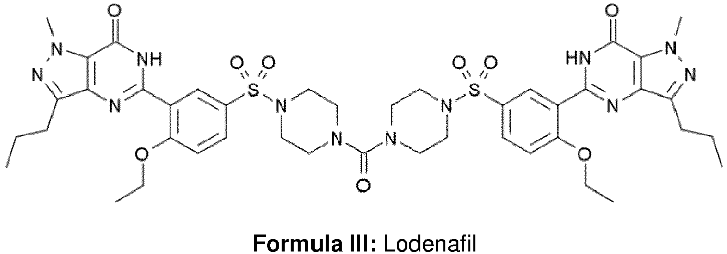

Mirodenafil is a PDE5 inhibitor used in the ED treatment and sold under the brand name of Mvix®. It is also found in the market under the brand name of Mvix S®. Its chemical name is 5-Ethyl-3,5-dihydro-2-[5-([4-(2-hidroxyethyl)-1-piperazinyl]sulfonyl)-2-propoxyiphenyl]-7-propyl-4H-pyrrole[3,2-d]pyrimidine-4-on. Its chemical structure is given in Formula IV.

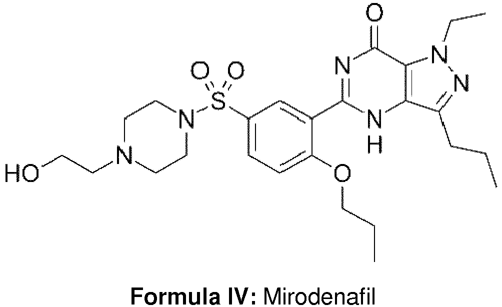

Sildenafil is a PDE5 inhibitor used in the treatment of ED and pulmonary arterial hypertension and is sold under the brand name of Viagra® and Revatio®. Its chemical name is 1-[4-ethoxy-3-(6,7-dihydro-1-methy-7-oxo-3-propyl-1H-pyrrazole[4,3-d]pyrimidine-5-yl)phenylsulfonyl]-4-methylpiperazine. Its chemical structure is shown in Formula V.

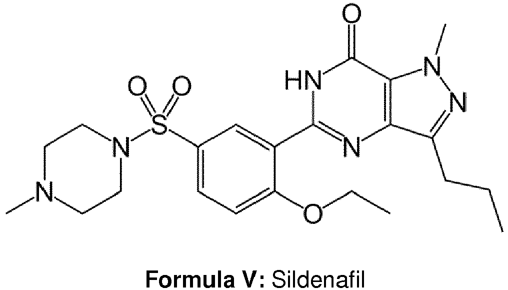

Tadalafil is a PDE5 inhibitor used in treatment of ED and PAH. It has a longer half life compared to other PDE5 inhibitors (mean 17,5 hours). Its chemical name is (6R-trans)-6-(1,3-benzodioxol-5-yl)- 2,3,6,7,12,12a-hexahydro-2-methyl-pyrazine [1', 2':1,6] pyrido[3,4-b]indol-1,4-dion. Its chemical structure is shown in Formula VI.

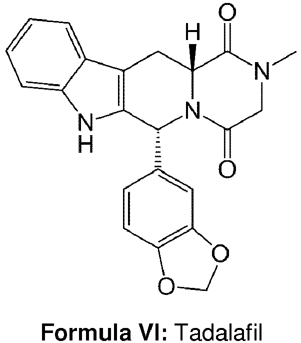

Vardenafil is a PDE5 inhibitor used in the ED treatment sold under the brand name of Levitra®. It is also sold in the form of coated tablet form under the brand name of Staxyn®. Its chemical name is 4-[2-Ethoxy-5-(4-ethylpiperazine-1-yl)sulfonyl-fenyl]-9-methyl-7-propyl-3,5,6,8-tetrazbicyclo[4.3.0]nona-3,7,9-triene-2-on. Its chemical structure is shown in Formula VII.

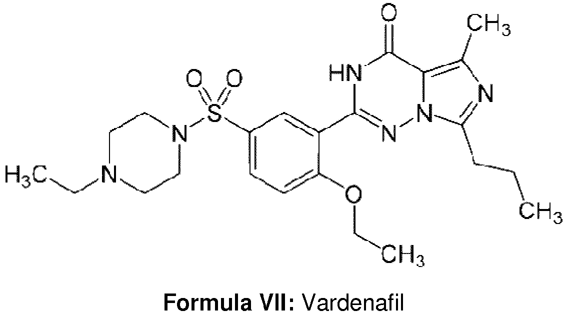

Udenafil is a PDE5 inhibitor used in the ED treatment sold under the brand name of Zydena®. Its chemical name is 3-(1-methyl-7-oxo-3-propyl-4,7-dihydro-1H-pyrrazole[4,3-d]pyrimidine-5-yl)-N-[2-(1-methylpyrrolidine-2-yl)ethyl]-4-propoxybenzenesulfonamide. Its chemical structure is shown in Formula VIII.

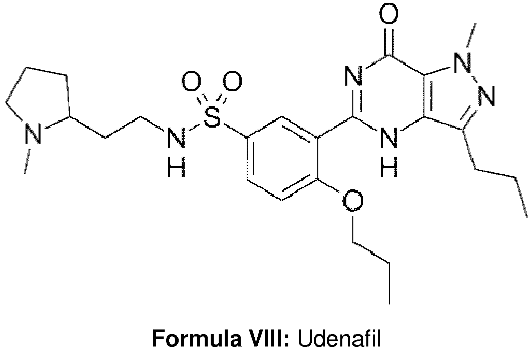

It is known that PDE5 inhibitors poorly disintegrate in water. Therefore, they have a low and inconsistent bioavailability. To solve this problem, several methods have been employed. For example, tadalafil (although disintegrates poorly in water) can disintegrate in organic solvents such as dimethylformamide and dimethylsulfoxide. In the patent No EP1200091 B1, hydrophilic solvents such as polyethylene glycol 400, propylene glycol, and glycofurole were used for the tadalafil which poorly disintegrated in water. In this patent, tadalafil disintegrated in hydrophilic solvents was packed in soft capsules.

Various formulations and methods are already known to prepare formulations disintegrated orally. However, treatment compliance with orally disintegrated formulations compared with conventional solid dosage forms such as capsules and tablets have gained importance. This subject is especially important for those who have difficulty in swallowing. In addition, one needs to ingest liquids with many drugs with resulting gastric volume increase which increases the likelihood of nausea and vomiting. The main advantage of orally disintegrating dosage forms is probably the rapid disintegration of solid dosage forms in oral cavity to form a solution or suspension without the need of fluid ingestion. By this way, it is sufficient to administer the dosage form immediately before a patient needs it. Furthermore, absorption of hydrophobic PDE5 inhibitors is low in stomach due to their poor disintegration in gastric fluid. Therefore, an orally disintegrating dosage form is advantageous in administration of drugs containing PDE5 inhibitors and dapoxetine and it increases treatment compliance along with recommended pharmaceutical therapies.

Besides, the orally disintegrating dosage form is one of the advantageous methods to deliver these drugs to such patients. By administering the orally disintegrating dosage forms, faster absorption of the drug occurs through buccal mucosa and it may reduce the first pass effect leading to better efficacy of the drug. This dosage form enhances the clinical effects of some drugs by leading to increase in the bioavailability and a reduction in the side effects because of avoidance offirst-pass liver metabolism.

It is clear to develop orally disintegrating compositions are difficult because of a number of different reasons. A satisfactory orally disintegrating dosage form needs to meet a number of requirements. Firstly, a disintegration in oral cavity earlier than intended may create some problems due to unpleasant taste of the active substance. Furthermore, these compositions should be highly porous and not very hard. These porous compositions tend to be very sensitive to moisture. As a result, they may have some stability problems. Lastly, an orally-disintegrated composition of suitable organoleptic and pharmacokinetic properties must also be manufactured commercially useful rates and yields.

To fulfill all these requirements, the formulation for a specific drug needs to be adapted in particular by a careful selection of excipients used. The selected excipients, however, may led to formulations which are not bioavailable to the corresponding conventional dosage forms. Therefore, they have to be chosen very carefully. In addition, precautions have to be taken at the preparation, packaging, handling, and storing of the finished dosage forms of orally disintegrating compositions since they tend to be both hygroscopic and friable.

Thus, there is a need of an orally disintegrating composition containing dapoxetine and a PDE5 inhibitor and a process to prepare such compositions. Further advantages and embodiments of the present invention will become apparent from the following description.

### Detailed description of the invention

The main object of the present invention is, by employing appropriate excipients, to prepare an orally disintegrating composition of dapoxetine or a pharmaceutically acceptable salt thereof with a PDE5 inhibitor or a pharmaceutically acceptable salt thereof beneficial in treatment of erectile dysfunction and related symptoms which overcomes the above-mentioned problems.

Another object of the present invention is to develop a simple, cost-effective, and timesaving process to prepare an orally disintegrating formulation of dapoxetine or a pharmaceutically acceptable salt with a PDE5 inhibitor or a pharmaceutically acceptable salt thereof.

Yet another object of the present invention is to provide an orally disintegrating combination of dapoxetine or apharmaceutically accepted salt thereof with a PDE5 inhibitor or a pharmaceutically accepted salt thereof which has good mechanical strength enoughto be processed in high tabletting machines (for example, adequate rigidity and low friability) and shipped in low-cost packages.

According to another embodiment, the orally disintegrating composition comprises dapoxetine or a pharmaceutically accepted salt thereof in the ratio of 1.0 to 30.0% and a PDE5 inhibitor or a pharmaceutically accepted salt thereof in the ratio of 1.0 to 40.0% by total composition weight.

Another object of the invention is to prepare an orally disintegrating composition containing a combination of dapoxetine or a pharmaceutically acceptable salt thereof with a PDE5 inhibitor or a pharmaceutically acceptable salt thereof, which has adequate bioavailability and is stable during shelf life. In line with this purpose, in the present invention, unexpected benefits were obtained with this orally disintegrating composition which was comparable with available conventional solid dosage forms.

The appropriate PDE5 inhibitor used in orally disintegrating compositions is selected from among the group comprising avanafil, lodenafil, mirodenafil, sildenafil, tadalafil, vardenafil, and udenafil; the preferred PDE5 inhibitor is tadalafil.

Each PDE5 inhibitor has a different onset and duration of effect. After the oral administration, the onset of action of sildenafil is 1 hour whereas it is 15 minutes for tadalafil. The duration of effect of sildenafil is 4 hours whereas tadalafil may be effective as long as 36 hours. Tadalafil is preferred in situations where a rapid onset of action is desirable.

Orally disintegrating compositions of the present invention further comprise one or more dispersing agents as listed in the following description.

According to one embodiment, the amount of dispersing agent is 15-40% by weight of the total composition, and said amount makes it possible to significantly improve compressibility, reduce friability and achieve a substantial reduction in disintegration time. Higher quantities may have negative mechanichal strength of the formula and lower quantities may worsen the disintegration time.

The dispersing agent mentioned above is selected from the group consisting of calcium silicate, heavy magnesium carbonate, cross-linked povidone, carboxymethyl cellulose calcium, magnesium aluminium silica, sodium dodesyl sulfate, sodium carboxymethly cellulose, croscarmellose sodium, docusate sodium, low-substitute hydroxypropylcellulose, a mixture of microcrystalline cellulose and guar glue (Avicel CE-15); poloxamere or mixtures thereof. It is selected preferably from the group comprising calcium silicate, heavy magnesium carbonate, crosspovidon or mixtures thereof. Dispersing agent of the present invention is preferably calcium silicate; mean particle size of calcium silicate is less than 50 microns, preferably less than 20 microns, and more preferably less than 10 microns.

According to this regulation of the invention, orally disintegrating composition comprises calcium silicate, heavy magnesium carbonate, crosspovidon or mixtures thereof in the ratio of 15.0-40.0% by the total composition weight and the formulation disintegrates in oral cavity in less than 60 seconds, preferably less than 30 seconds, and more preferably less than 20 seconds.

The interval selected for the ratio of the dispersing agents in the composition to the dapoxetine has been unexpectedly found to produce an orally disintegrating composition overcoming the above-mentioned disadvantages of orally disintegrating compositions. The ratio of total weight of the dispersing agents to the total weight of dapoxetine is between 0.5:1 and 5:1.

It has been unexpectedly discovered that use of a binder in addition to the dispersing agent in the orally disintegrating composition of a combination of dapoxetine or a pharmaceutically acceptable salt thereof with a PDE5 inhibitor or a pharmaceutically acceptable salt thereof has a synergistic effect on dinsintegration time. The appropriate binder is selected from the group comprising cellulose derivatives such as pullulan, starch, polyvinylpyrrolidone (povidone), gelatin, sugars, glucose, natural glues, gums, synthetic celluloses, polymethacrylate, hydroxypropyl methyl cellulose, microcrystalline cellulose, hydroxypropyl cellulose, carboxy methyl cellulose, methyl cellulose. Polyvinylpyrrolidone (povidone), pullulan, microcrystalline cellulose or mixtures thereof are preferably used as binder. In compliance with this object of the invention, the orally disintegrating compositions comprise one or more binders in the ratio of 0.1 to 20.0%, and preferably in the ratio of 0.2-15.0% by the total composition weight.

Compositions suitable for the present invention may be in the form of a tablet. Compositions appropriate for the invention are preferably in the form of an orally disintegrating tablet.

Another object of the present invention is to develop orally disintegrating tablet formulations with optimal mechanical resistance. The present invention meets this demand and explains tablet formulations rapidly disintegrated in oral cavity. These tablet compositions have a pleasant mouth feel and good mechanical strength. These tablets are robust (e.g., low friability, adequate hardness) enough to be processed in high speed tableting machines and shipped in low cost packages, and at the same time retain rapid disintegration or dissolution properties. These orally disintegrating compositions are bioavailable in correspondence with the conventional solid dosage formulations and stable throughout the shelf-life.

According to another object of the present invention, the hardness of the orally disintegrating tablet is between 5 N and 100 N and preferably 20 N and 45 N, and its friability is below 1.0%.

It is known that, to develop orally disintegrating compositions are difficult because of several different reasons. An appropriate orally disintegrating dosage form should meet some requirements. First, it should rapidly disintegration oral cavity. On the other hand, release or disintegration of the active substance earlier than intended may pose some problems due to generally unpleasant taste of the active agent. In addition, compositions should be highly porous but not too hard. These porous compositions are highly sensitive to moisture. As a result, they may have some stability problems.

However, orally disintegrating tablets should be pleasant-tasting to be pharmaceutically acceptable, e.g. they should possess acceptable organoleptic properties such as giving a pleasant sensation or taste to mouth because orally disintegrating tablets are designed to disintegrate rapidly in oral cavity without remaining substantial amounts of the active substances. In addition, the orally disintegrating tablets should possess acceptable pharmacokinetic properties and bioavailability to provide desired therapeutic effect. In contrast, composition substances in the formulation supporting rapid release may cause unpleasant sensation or taste in mouth. Lastly, orally disintegrating compositions with suitable organoleptic and pharmacokinetic properties should be manufactured at commercially beneficial rates and yields.

Consequently, an acceptable orally-disintegrating composition should balance these contradictory characteristics to obtain a composition possessing acceptable pharmacokinetic properties, having a pleasant taste (for example, taste-masked), and rapid disintegration.

To fulfill all these requirements, a formulation for a specific drug particularly necessitates careful selection of the excipients. However, the selected excipients could yield formulations with no good bioavailability for the corresponding conventional dosage forms. Therefore, they should be selected very carefully.

The invention contains at least one pharmaceutically acceptable excipient selected from the group comprising of fillers, lubricants, sweeteners, aromatic agents, or coloring agents.

Suitable fillers may include, but not limited to, mannitol, spray-dried mannitol; polysaccharides, dibasic calcium phosphate dihydrate, lactose, sugars, sorbitol; mixture of mannitol, polyplasdone, and ciolide (Phramaburst); mixture of mannitol, crospovidone, and polyvinyl acetate (Ludiflash); isomalt, sucrose, and inorganic salts such as calcium salts, and their mixtures; they preferably contain mannitol. Orally disintegrating tablet formulations according to the invention comprise fillers in the ratio of 2.0 to 90.0% and preferably 10.0-75.0% by the total tablet weight.

Suitable lubricants may include, but not limited to, sodium stearyl fumarate, magnesium stearate, polyethylene glycol, stearic acid, metal stearates, boric acid, sodium chloride benzoate and acetate, sodium or magnesium lauryl sulfate, and mixtures thereof; the preferred lubricant is sodium stearyl fumarate. Sodium stearyl fumarate is highly effective lubricant, being less hydrophobic than magnesium stearate and has a less retarding effect on tablet disintegration compared to magnesium stearate. In addition, sodium stearyl fumarate does not have problems due to overblending observed in magnesium stearate. Consistent with this object of the invention, the orally disintegrating tablet formulations comprise sodium stearyl fumarate in the ratio of 0.1 to 10.0% and preferably 0.2 to 5.0% by the total tablet weight.

Suitable sweeteners may include, but not limited to, sucralose, acesulfame-K, aspartame, saccharine or sodium and calcium salts of saccharine, sodium cyclamate, sucrose, fructose, glucose, sorbitol, and mixtures thereof; the preferred sweetener is sucralose. In the present invention, sucralose is present in the ratio of 0.01 to 5.0% and preferably 0.05 to 2.0% by the total tablet weight.

Suitable aromatic agents may include, but not limited to, fruit aromas such as orange, banana, strawberry, cherry, wild cherry, and lemon, other aromas including cordamom, anasone, mint, menthol, vanillin, and ethyl vanillin, and mixtures thereof; the preferred aromatic agent is selected from fruit aromas, such as orange aroma. According to the invention, the aromatic agent is in the ratio of 0.05-5.0% by the total tablet weight.

Formulation in the present invention can be manufactured with direct compression method and also with the following methods:
Process I: Spray drying: Active substances are separately disintegrated or dispersed with a binder in a polymer, and then they are subjected to spray-drying process. The obtained particles are mixed with other auxiliary products, a lubricant is added to the mixture, the mixture is blended for a short period, and tablet press process is performed.
Process II: Coacervation: Active substances and polymer are disintegrated with an appropriate solvent. The obtained solution is blended at a certain speed and temperature while a solvent is added to make polymer and active substance precipitate. When necessary, pH regulating substances are used to facilitate precipitation. Polymers coat the active substance while they precipitate and spherical microparticles are obtained during the process. The obtained coated particles are dried and sifted to obtain appropriate particle size distribution. Coated particles are mixed with other excipients in the formula, a lubricant is then added to the mixture, and tablet press process is performed after a short period of blending.
Process III: Hot melt extrusion: Active substances are melted together with a meltable polymer, pass through an extruder, subjected to spherization, and sieved to provide a suitable particle size distribution. The obtained particles are mixed with other excipients in the formulation, a lubricant added to the mixture, and tablet press process is performed after a short period of blending.
Process IV: Dual layer tablet: Active substances are mixed with dispersing agents and filler; granulated with alcoholic/hydroalcoholic binding (polymer) solution, dried, and significantly, sifted. The remaining auxialiary substances in the formulation are separately added to both phases, blended, lubricants are then separately added, and press process is performed in bilayer tablet machine after a short period of blending.

In addition to above-mentioned processes, orally disintegrating tablets can also be produced via pellet coating method. In this method, sugar pellets should also be used in addition to excipients. Hydroalcoholic/alcoholic solutions with PVP of active substances tadalafil and dapoxetine are prepared and sugar pellets are separately coated with these preparations. Polymetachrylate; alcoholic/hydroalcoholic solutions are prepared and sugar pellets containing tadalafil and dapoxetine are separately coated. The obtained tadalafil and dapoxetine pellets are mixed with other excipients in the formulation, a lubricant is than added to the mixture and tablet press process is performed after blending for a short period.

According to a different view, the present invention shows that the disintegration rate of the tablet can be altered substantially by changing the shape and size of the tablet. In general, as the tablet becomes thinner and more porous the orally disintegrating composition will be weakened more rapidly when exposed to saliva because process of disintegration takes place after all surface area of the tablet is moistened by the capillary effect. In addition, any shape maximizing contact surface with saliva can remarkably shorten the period of the disintegration.

The preferred shape of the orally disintegrating tablet composition of this invention may be a disk, a circle, a sphere, a globe, a gobbet, a rod, a polygon, and ellipse. The preferred shape of the tablet is a flat circle.

The invention is explained in detail with the following examples. The examples do not limit the scope of the invention and should be considered in the context of the description detailed above.

### Examples

### Example 1 - Orally disintegrating tablets containing dapoxetine and tadalafil

| **Ingredients** | **Amount %** |
|---|---|
| Tadalafil | 6.67 |
| Dapoxetine | 10.00 |
| Calcium Silicate | 20.13 |
| Mannitol | 50.00 |
| Polyvinyl pyrrolidone | 1.00 |
| Crospovidone | 10.00 |
| Sucralose | 0.20 |
| Orange aroma | 1.00 |
| Sodium stearyl fumarate | 1.00 |

### Manufacturing method: Direct compression method

Tadalafil, dapoxetine, mannitol, and crosspovidon are mixed. Polyvinyl pyrrolidone, calcium silicate, sucralose, and orange aroma are added to this mixture and the mixture is blended until a homogenous mixture is obtained. Sodium stearyl fumarate is added to the final mixture and after blending for a short period, tablet compression process is performed.

### Example 2 - Orally disintegrating tablets containing dapoxetine and sildenafil

| **Ingredients** | **Amount%** |
|---|---|
| Dapoxetine | 15.00 |
| Sildenafil | 25.00 |
| Mannitol | 29.85 |
| Heavy magnesium carbonate | 17.13 |
| Polyvinyl pyrrolidone | 1.00 |
| Sucralose | 0.20 |
| Orange aroma | 1.00 |
| Crospovidone | 10.00 |
| Sodium stearyl fumarate | 1.00 |

### Manufacturing method:

Sildenafil, dapoxetine, mannitol, and a part of crospovidone are mixed, granulated with alcoholic/hydroalcoholic polyvinyl pyrrolidone solution, dried, and sifted. Calcium silicate, the remaining part of crosspovidon, sucralose, and orange aroma are added to this mixture and blended until a homogenous mixture is obtained. Sodium stearyl fumarate is added to the final mixture and tablet compression process is performed after mixing for a while.

Example 3 will be manufactured with the same method.

### Example 3 - Orally disintegrating tablets containing dapoxetine and vardenafil

| **Ingredients** | **Amount %** |
|---|---|
| Dapoxetine | 20.00 |
| Vardenafil | 6.67 |
| Mannitol | 43.00 |
| Calcium silicate | 17.13 |
| Polyvinyl pyrrolidone | 1.00 |
| Sucralose | 0.20 |
| Orange aroma | 1.00 |
| Crospovidone | 10.00 |
| Sodium stearyl fumarate | 1.00 |

### Example 4 - Orally disintegrating tablets containing dapoxetine and avanafil

| **Ingredients** | **Amount %** |
|---|---|
| Dapoxetine | 20.00 |
| Avanafil | 16.66 |
| Mannitol | 41.30 |
| Heavy magnesium carbonate | 17.83 |
| Pullulan | 1.00 |
| Polyvinyl alcohol | 1.00 |
| Sucralose | 0.20 |
| Orange aroma | 1.00 |
| Sodium stearyl fumarate | 1.00 |

### Manufacturing method:

Avanafil, dapoxetine and a part of mannitol is mixed, granulated with alcoholic/hydroalcoholic pullulan solution, dried, and sifted. The obtained granules are coated with alcoholic/hydroalcoholic polyvinyl alcohol solution in a fluidized-bed system. The remaining part of mannitol, heavy magnesium carbonate, sucralose, and orange aroma is added to this mixture and blended until a homogenous mixture is obtained. Sodium stearyl fumarate is added to the final mixture and tablet compression process is performed after mixing for a while.

Examples 5 and 6 will be manufactured with the same method.

### Example 5 - Orally disintegrating tablets containing dapoxetine and lodenafil

| **Ingredients** | **Amount** |
|---|---|
| Dapoxetine | 10.00 |
| Lodenafil | 6.67 |
| Mannitol | 56.47 |
| Calcium silicate | 22.67 |
| Pullulan | 1.00 |
| Polyvinyl alcohol | 1.00 |
| Sucralose | 0.20 |
| Orange aroma | 1.00 |
| Sodium stearyl fumarate | 1.00 |

### Example 6 - Orally disintegrating tablets containing dapoxetine and tadanafil

| **Ingredients** | **Amount %** |
|---|---|
| Dapoxetine | 20.00 |
| Tadalafil | 6.67 |
| Mannitol | 49.40 |
| Heavy magnesium carbonate | 19.73 |
| Pullulan | 1.00 |
| Polyvinyl alcohol | 1.00 |
| Sucralose | 0.20 |
| Orange aroma | 1.00 |
| Sodium stearyl fumarate | 1.00 |

### Example 7 - Orally disintegrating tablets containing dapoxetine and mirodenafil

| **Ingredients** | **Amount %** |
|---|---|
| Dapoxetine | 20.00 |
| Mirodenafil | 16.66 |
| Mannitol | 10.94 |
| Microcrystalline cellulose | 5.73 |
| Avicel CE-15 | 9.00 |
| Calcium silicate | 19.47 |
| Polyvinyl alcohol | 1.00 |
| Crospovidone | 15.00 |
| Sucralose | 0.20 |
| Orange aroma | 1.00 |
| Sodium stearyl fumarate | 1.00 |

### Manufacturing method:

Mirodenafil and dapoxetine are separately coated with alcoholic/hydroalcoholic polyvinyl alcohol solution. Microcrystalline cellulose, mannitol and crosspovidone, calcium silicate, Avicel-CE 15, sucralose, and orange aroma are added to this mixture and blended until a homogenous mixture is obtained. Sodium stearyl fumarate is added to the final mixture and tablet compression process is performed after blending for a while.

Examples 8 and 9 will be manufactured with the same method.

### Example 8 - Orally disintegrating tablets containing dapoxetine and udenafil

| **Ingredients** | **Amount %** |
|---|---|
| Dapoxetine | 10.00 |
| Udenafil | 33.33 |
| Mannitol | 2.33 |
| Microcrystalline cellulose | 7.67 |
| Avicel CE-15 | 9.00 |
| Calcium silicate | 19.47 |
| Polyvinyl alcohol | 1.00 |
| Crospovidone | 15.00 |
| Sucralose | 0.20 |
| Orange aroma | 1.00 |
| Sodium stearyl fumarate | 1.00 |

### Example 9 - Orally disintegrating tablets containing dapoxetine and tadalafil

| **Ingredients** | **Amount%** |
|---|---|
| Dapoxetine | 20.00 |
| Tadalafil | 6.67 |
| Mannitol | 19.00 |
| Microcrystalline cellulose | 7.67 |
| Avicel CE-15 | 9.00 |
| Calcium silicate | 19.47 |
| Polyvinyl alcohol | 1.00 |
| Crospovidone | 15.00 |
| Sucralose | 0.20 |
| Orange aroma | 1.00 |
| Sodium stearyl fumarate | 1.00 |

### Example 10 - Orally disintegrating tablets containing dapoxetine and sildenafil

| **Ingredients** | **Amount %** |
|---|---|
| Dapoxetine | 5.00 |
| Sildenafil | 25.00 |
| Sugar pellet | 20.00 |
| Eudragit E100(polymethyl metachrylate) | 10.00 |
| Mannitol | 12.24 |
| Microcrystalline cellulose | 4.56 |
| Calcium silicate | 10.00 |
| Polyvinyl pyrrolidone (PVP) | 1.00 |
| Crospovidone | 10.00 |
| Sucralose | 0.20 |
| Orange aroma | 1.00 |
| Sodium stearyl fumarate | 1.00 |

- Hydroalcoholic/alcoholic solutions with PVP of sildenafil and dapoxetine active substances are prepared and sugar pellets are separately coated with these preparations.
- Polymetachrylate: alcoholic/hydroalcoholic solutions thereof are prepared and sugar pellets containing sildenafil and dapoxetine are separately coated.
- The obtained sildenafil and dapoxetine pellets are mixed with mannitol, microcrystalline cellulose, calcium silicate, crospovidone, sucralose, and orange aroma, then sodium stearyl fumarate is added to the mixture and tablet compression process is performed after blending for a while.

### Example 11 - Orally disintegrating tablets containing dapoxetine and tadalafil

| **Ingredients** | **Amount %** |
|---|---|
| Dapoxetine | 10.00 |
| Tadalafil | 6.67 |
| Sugar pellet | 20.00 |
| Eudragit E100(polymethyl methacrylate) | 10.00 |
| Mannitol | 21.47 |
| Microcrystalline cellulose | 8.67 |
| Calcium silicate | 10.00 |
| Polyvinyl pyrrolidone (PVP) | 1.00 |
| Crospovidone | 10.00 |
| Sucralose | 0.20 |
| Orange aroma | 1.00 |
| Sodium stearyl fumarate | 1.00 |

- Hydroalcoholic/alcoholic solutions with PVP of tadalafil and dapoxetine active substances are separately prepared and sugar pellets are separately coated with these preparations.
- Polymetachrylate; alcoholic/hydroalcoholic solutions are prepared and sugar pellets containing tadalafil and dapoxetine are separately coated.
- The obtained tadalafil and dapoxetine pellets are mixed with mannitol, microcrystalline cellulose, calcium silicate, crospovidone, sucralose, and orange aroma, then sodium stearyl fumarate is added to the mixture and tablet compression process is performed after blending for a while.

## Claims

1. An orally disintegrating composition comprising, dapoxetine or a pharmaceutically acceptable salt thereof, a PDE5 inhibitor or a pharmaceutically acceptable salt thereof and at least one dispersing agent;
said dispersing agent being present in an amount of from 15 to 40% by weight of the total composition, and
said dispersing agent is selected from the group consisting of calcium silicate, heavy magnesium carbonate, cross-linked povidone, carboxymethyl cellulose calcium, magnesium aluminum silica, sodium dodesyl sulfate, sodium carboxymethyl cellulose, croscarmellose sodium, docusate sodium, low-substituted hydroxypropylcellulose, a mixture of microcrystalline cellulose and guar glue, poloxamer and mixtures thereof.

2. An orally disintegrating composition according to claim 1, wherein; dapoxetine or a pharmaceutically acceptable salt thereof is present in an amount of 1.0 to 30.0% by weight of the total composition and a PDE5 inhibitor or a pharmaceutically acceptable salts thereof is present in an amount of 1.0 to 40.0% by weight of the total composition.

3. An orally disintegrating composition according to claims 1 and 2, wherein, the PDE5 inhibitor is selected from the group consisting of avanafil, lodenafil, mirodenafil, sildenafil, tadalafil, vardenafil, and udenafil.

4. An orally disintegrating composition according to claim 1, wherein the dispersing agent is preferably selected from the group consisting of calcium silicate, heavy magnesium carbonate, crospovidone, and mixtures thereof.

5. An orally disintegrating composition according to claim 4, wherein the mean particle size of calcium silicate that is used as a dispersing agent is less than 50 microns, preferably less than 20 microns, and more preferably less than 10 microns.

6. An orally disintegrating composition according to claims 1 to 5, wherein the weight ratio of the dispersing agents to dapoxetine is in the range of 0.5:1 to 5:1.

7. An orally disintegrating composition according to claim 1, further comprises one or more binders.

8. An orally disintegrating composition according to claim 7, wherein the amount of the binder is 0.1 to 20% by weight of the total composition, preferably it is 0.2 to 15% by weight of the total composition.

9. An orally disintegrating composition according to claims 7 and 8, wherein it comprises pullulan, starch, polyvinylpyrrolidone (povidone), gelatin, sugars, glucose, natural glues, gums, synthetic celluloses, polymethacrylate, hydroxypropyl methyl cellulose, microcrystalline cellulose, hydroxypropyl cellulose, carboxy methyl cellulose, methyl cellulose, cellulose derivatives, or their mixtures as binders.

10. An orally disintegrating composition according to claim 9, wherein the binder is preferably selected from the group consisting of polyvinylpyrrolidone (povidone), pullulan, microcrystalline cellulose and mixtures thereof.

11. An orally disintegrating composition according to claims 1 to 10, wherein the composition is in the form of a tablet.

12. An orally disintegrating tablet according to claim 11, wherein the hardness of the tablet is between 5 N to 100 N and preferably 20 N to 45 N and friability is below 1.0%.

13. An orally disintegrating tablet according to any of the previous claims, wherein at least one pharmaceutically acceptable excipient is selected from the group consisting of lubricants, fillers, sweeteners, aromatic agents or coloring agents.

14. An orally disintegrating tablet according to claim 13, wherein it comprises mannitol, spray-dried mannitol, polysaccharides, dibasic calcium phosphate dihydrate, lactose, sugars, sorbitol, isomalt, sucrose, inorganic salts such as calcium salts and mixtures thereof as fillers.

15. An orally disintegrating tablet according to claim 14, wherein the filler is mannitol.

16. An orally disintegrating tablet according to claims 14 and 15, wherein the filler is present in an amount of 2.0 to 90.0% by weight of the total composition, preferably 10.0 to 75.0 % by weight of total the total composition.

17. An orally disintegrating tablet according to claim 13, wherein it comprises sodium stearyl fumarate, magnesium stearate, polyethylene glycol, stearic acid, metal stearats, boric acid, sodium chloride benzoate and acetate, sodium or magnesium lauryl sulfate or mixtures thereof as lubricant.

18. An orally disintegrating tablet according to claim 17, wherein the lubricant is preferably sodium stearyl fumarate.

19. An orally disintegrating tablet according to claim 18, wherein sodium stearyl fumarate is present in an amount of 0.1 to 10.0% by weight of the total composition, preferably 0.2 to 5.0 % by weight of total composition.

20. An orally disintegrating tablet according to claim 13, wherin sweeteners are selected from sucralose, acesulfame-K, aspartam, saccharine or saccharine sodium and calcium salts, sodium cyclamate, sucrose, fructose, glucose, sorbitol, and their mixtures; the preferred sweetener is sucralose.

21. An orally disintegrating tablet according to claim 20, wherein sucralose is present in an amount of 0.01 to 5.0% by weight of the total composition, preferably 0.05 to 2.0 % by weight of total composition.

22. An orally disintegrating tablet according to claim 13, wherein aromatic agents are selected from fruit aromas such as orange, banana, strawberry, cherry, wild cherry, lemon, and other aromas such as cardamom, anasone, mint, menthol, vanillin, and ethyl vanillin, and their mixtures, the preferred aromatic agent is selected from fruit aromas, such as orange aroma.

23. An orally disintegrating tablet according to claim 22, wherein the aromatic agent is present in an amount of 0.05 to 5.0% by weight of the total composition.

## Patentansprüche

1. Oral sich auflösende Zusammensetzung, umfassend Dapoxetin oder ein pharmazeutisch akzeptables Salz hiervon, einen PDE5-Inhibitor oder ein pharmazeutisch akzeptables Salz hiervon und mindestens ein Dispergiermittel,
wobei das Dispergiermittel in einer Menge von 15 bis 40 %, bezogen auf das Gewicht der gesamten Zusammensetzung, vorhanden ist und
das Dispergiermittel aus der Gruppe ausgewählt ist, die aus Calciumsilicat, schwerem Magnesiumcarbonat, vernetztem Povidon, Calciumcarboxymethylcellulose, Magnesium-Aluminium-Siliciumdioxid, Natriumdodecylsulfat, Natriumcarboxymethylcellulose, Natriumcroscarmellose, Docusat-Natrium, niedrig substituierter Hydroxypropylcellulose, einem Gemisch von mikrokristalliner Cellulose und Guarkleber, Poloxamer und Gemischen hiervon besteht.

2. Oral sich auflösende Zusammensetzung gemäß Anspruch 1, wobei Dapoxetin oder ein pharmazeutisch akzeptables Salz hiervon in einer Menge von 1,0 bis 30,0 %, bezogen auf das Gewicht der gesamten Zusammensetzung, vorhanden ist und ein PDE5-Inhibitor oder ein pharmazeutisch akzeptables Salz hiervon in einer Menge von 1,0 bis 40,0 %, bezogen auf das Gewicht der gesamten Zusammensetzung, vorhanden ist.

3. Oral sich auflösende Zusammensetzung gemäß den Ansprüchen 1 und 2, wobei der PDE5-Inhibitor aus der Gruppe ausgewählt ist, die aus Avanafil, Lodenafil, Mirodenafil, Sildenafil, Tadalafil, Vardenafil und Udenafil besteht.

4. Oral sich auflösende Zusammensetzung gemäß Anspruch 1, wobei das Dispergiermittel vorzugsweise aus der Gruppe ausgewählt ist, die aus Calciumsilicat, schwerem Magnesiumcarbonat, Crospovidon und Gemischen hiervon besteht.

5. Oral sich auflösende Zusammensetzung gemäß Anspruch 4, wobei die mittlere Partikelgröße von Calciumsilicat, welches als Dispergiermittel verwendet wird, weniger als 50 Mikrometer, vorzugsweise weniger als 20 Mikrometer und stärker bevorzugt weniger als 10 Mikrometer beträgt.

6. Oral sich auflösende Zusammensetzung gemäß den Ansprüchen 1 bis 5, wobei das Gewichtsverhältnis der Dispergiermittel zu Dapoxetin in einem Bereich von 0,5:1 bis 5:1 liegt.

7. Oral sich auflösende Zusammensetzung gemäß Anspruch 1, die des Weiteren ein oder mehrere Bindemittel umfasst.

8. Oral sich auflösende Zusammensetzung gemäß Anspruch 7, wobei die Menge des Bindemittels 0,1 bis 20 %, bezogen auf das Gewicht der gesamten Zusammensetzung, vorzugsweise 0,2 bis 15 %, bezogen auf das Gewicht der gesamten Zusammensetzung, beträgt.

9. Oral sich auflösende Zusammensetzung gemäß Anspruch 7 und Anspruch 8, wobei diese Pullulan, Stärke, Polyvinylpyrrolidon (Povidon), Gelatine, Zucker, Glucose, natürliche Kleber, Gummis, synthetische Cellulosen, Polymethacrylat, Hydroxypropylmethylcellulose, mikrokristalline Cellulose, Hydroxypropylcellulose, Carboxymethylcellulose, Methylcellulose, Cellulosederivate, oder deren Gemische als Bindemittel umfasst.

10. Oral sich auflösende Zusammensetzung gemäß Anspruch 9, wobei das Bindemittel vorzugsweise aus der Gruppe ausgewählt ist, die aus Polyvinylpyrrolidon (Povidon), Pullulan, mikrokristalliner Cellulose und Gemischen hiervon besteht.

11. Oral sich auflösende Zusammensetzung gemäß den Ansprüchen 1 bis 10, wobei die Zusammensetzung die Form einer Tablette aufweist.

12. Oral sich auflösende Tablette gemäß Anspruch 11, wobei die Härte der Tablette zwischen 5 N bis 100 N und vorzugsweise 20 N bis 45 N beträgt und die Brüchigkeit unter 1,0 % beträgt.

13. Oral sich auflösende Tablette gemäß einem der vorhergehenden Ansprüche, wobei mindestens ein pharmazeutisch akzeptabler Hilfsstoff aus der Gruppe ausgewählt ist, die aus Gleitmitteln, Füllstoffen, Süßungsmittel, Aromamitteln oder Farbmitteln besteht.

14. Oral sich auflösende Tablette gemäß Anspruch 13, wobei diese Mannitol, sprühgetrocknetes Mannitol, Polysaccharide, dibasisches Calciumsphosphatdihydrat, Lactose, Zucker, Sorbitol, Isomalt, Saccharose, anorganische Salze, wie beispielsweise Calciumssalze, und Gemische hiervon als Füllstoffe umfasst.

15. Oral sich auflösende Tablette gemäß Anspruch 14, wobei der Füllstoff Mannitol ist.

16. Oral sich auflösende Tablette gemäß den Ansprüchen 14 und 15, wobei der Füllstoff in einer Menge von 2,0 bis 90,0 %, bezogen auf das Gewicht der gesamten Zusammensetzung, vorzugsweise 10,0 bis 75,0 %, bezogen auf das Gewicht der gesamten Zusammensetzung, vorhanden ist.

17. Oral sich auflösende Tablette gemäß Anspruch 13, wobei diese Natriumstearylfumarat, Magnesiumstearat, Polyethylenglycol, Stearinsäure, Metallstearate, Borsäure, Natriumchlorid-benzoat und -acetat, Natrium- oder Magnesiumlaurylsulfat oder Gemische hiervon als Gleitmittel umfasst.

18. Oral sich auflösende Tablette gemäß Anspruch 17, wobei das Gleitmittel vorzugsweise Natriumstearylfumarat ist.

19. Oral sich auflösende Tablette gemäß Anspruch 18, wobei Natriumstearylfumarat in einer Menge von 0,1 bis 10,0 %, bezogen auf das Gewicht der gesamten Zusammensetzung, vorzugsweise 0,2 bis 5,0 %, bezogen auf das Gewicht der gesamten Zusammensetzung, vorhanden ist.

20. Oral sich auflösende Tablette gemäß Anspruch 13, wobei die Süßungsmittel aus Sucralose, Acesulfam-K, Aspartam, Saccharin oder Saccharin-Natrium- und -Calciumsalzen, Natriumcyclamat, Saccharose, Fructose, Glucose, Sorbitol und deren Gemischen ausgewählt sind; wobei das bevorzugte Süßungsmittel Sucralose ist.

21. Oral sich auflösende Tablette gemäß Anspruch 20, wobei Sucralose in einer Menge von 0,01 bis 5,0 %, bezogen auf das Gewicht der gesamten Zusammensetzung, vorzugsweise 0,05 bis 2,0 %, bezogen auf das Gewicht der gesamten Zusammensetzung, vorhanden ist.

22. Oral sich auflösende Tablette gemäß Anspruch 13, wobei die Aromamittel aus Fruchtaromen, wie beispielsweise Orange, Banane, Erdbeere, Kirsche, Wildkirsche, Zitrone und weiteren Aromen, wie beispielsweise Kardamom, Anis, Minze, Menthol, Vanillin und Ethylvanillin sowie deren Gemischen ausgewählt sind, wobei das bevorzugte Aromamittel aus Fruchtaromen, wie beispielsweise Orangenaroma, ausgewählt ist.

23. Oral sich auflösende Tablette gemäß Anspruch 22, wobei das Aromamittel in einer Menge von 0,05 bis 5,0 %, bezogen auf das Gewicht der gesamten Zusammensetzung, vorhanden ist.

## Revendications

1. Une composition à désintégration orale comprenant la dapoxétine ou un sel pharmaceutiquement acceptable de celui-ci, un inhibiteur de la PDE5 ou un sel pharmaceutiquement acceptable de celui-ci, et au moins un agent dispersant ;
ledit agent dispersant étant présent en une quantité allant de 15 à 40% en poids de la composition totale, et
ledit agent dispersant est choisi dans le groupe constitué par le silicate de calcium, le carbonate de magnésium lourd, la povidone réticulée, le carboxyméthylcellulose calcium, l'aluminium silice magnésium, le sulfate de sodium dodesyl, la cellulose carboxyméthyle de sodium, la croscarmellose sodique, le docusate sodique, l'hydroxypropylcellulose à substitution basse, un mélange de cellulose microcristalline et de gomme guar, le poloxamère et leurs mélanges.

2. Composition à désintégration orale selon la revendication 1, dans lequel la dapoxétine ou d'un de ses sels pharmaceutiquement acceptables est présent en une quantité de 1,0 à 30,0% en poids de la composition totale, et un inhibiteur de la PDE5 ou un de ses sels pharmaceutiquement acceptables est présent en une quantité de 1,0 à 40,0% en poids de la composition totale.

3. Composition à désintégration orale selon les revendications 1 et 2, dans laquelle l'inhibiteur de la PDE5 est choisi dans le groupe constitué par avanafil, lodenafil, mirodenafil, sildenafil, le tadalafil, le vardénafil, et udenafil.

4. Composition à désintégration orale selon la revendication 1, dans laquelle l'agent dispersant est de préférence choisi dans le groupe constitué par le silicate de calcium, le carbonate de magnésium lourd, la crospovidone et leurs mélanges.

5. Composition se désintégrant par voie orale selon la revendication 4, dans laquelle la taille moyenne des particules de silicate de calcium qui est utilisé comme un agent de dispersion est inférieur à 50 microns, de préférence inférieure à 20 microns, et plus préférablement encore inférieure à 10 microns.

6. Composition à désintégration orale selon les revendications 1 à 5, dans laquelle le rapport pondéral des agents dispersants à la dapoxétine est dans la plage de 0,5 : 1 à 5 : 1.

7. Composition à désintégration orale selon la revendication 1, comprenant en outre un ou plusieurs liants.

8. Composition à désintégration orale selon la revendication 7, dans laquelle la quantité de liant est de 0,1 à 20% en poids de la composition totale, de préférence de 0,2 à 15% en poids de la composition totale.

9. Composition à désintégration orale selon les revendications 7 et 8, dans laquelle elle comprend le pullulane, l'amidon, la polyvinylpyrrolidone (povidone), la gélatine, les sucres, le glucose, les colles naturelles, des gommes, des celluloses synthétiques, le polyméthacrylate, l'hydroxypropyl méthyl cellulose, la cellulose microcristalline, l'hydroxypropylcellulose, la carboxyméthylcellulose, la méthylcellulose, les dérivés de cellulose, ou leurs mélanges en tant que liants.

10. Composition à désintégration orale selon la revendication 9, dans laquelle le liant est de préférence choisi dans le groupe constitué par la polyvinylpyrrolidone (povidone), le pullulane, la cellulose microcristalline et leurs mélanges.

11. Composition à désintégration orale selon les revendications 1 à 10, dans laquelle la composition se présente sous la forme d'un comprimé.

12. Comprimé à désintégration orale selon la revendication 11, dans lequel la dureté du comprimé est comprise entre 5 N et 100 N et de préférence 20 N à 45 N et la friabilité est inférieure à 1,0%.

13. Comprimé à désintégration orale selon l'une quelconque des revendications précédentes, dans lequel au moins un excipient pharmaceutiquement acceptable est choisi dans le groupe constitué par les lubrifiants, les charges, les édulcorants, les agents aromatiques ou les agents colorants.

14. Comprimé à désintégration orale selon la revendication 13, dans lequel il comprend du mannitol, du mannitol séché par atomisation, des polysaccharides, du phosphate de calcium dibasique dihydraté, du lactose, des sucres, du sorbitol, de l'isomalt, du saccharose, des sels inorganiques tels que des sels de calcium et leurs mélanges comme charges.

15. Comprimé à désintégration orale selon la revendication 14, dans lequel la charge est le mannitol.

16. Comprimé à désintégration orale selon les revendications 14 et 15, dans lequel la charge est présente en une quantité de 2,0 à 90,0% en poids de la composition totale, de préférence de 10,0 à 75,0% en poids du total de la composition totale.

17. Comprimé à désintégration orale selon la revendication 13, dans lequel il comprend du stéarylfumarate de sodium, du stéarate de magnésium, du polyéthylèneglycol, de l'acide stéarique, des stéarates métalliques, de l'acide borique, du benzoate et de l'acétate de chlorure de sodium, du laurylsulfate de sodium ou de magnésium ou des mélanges de ceux-ci comme lubrifiant.

18. Comprimé à désintégration orale selon la revendication 17, dans lequel le lubrifiant est de préférence le stéarylfumarate de sodium.

19. Comprimé à désintégration orale selon la revendication 18, dans lequel le stéarylfumarate de sodium est présent en une quantité de 0,1 à 10,0% en poids de la composition totale, de préférence de 0,2 à 5,0% en poids de la composition totale.

20. Comprimé à désintégration orale selon la revendication 13, dans lequel les édulcorants sont choisis parmi le sucralose, l'acésulfame-K, l'aspartam, la saccharine ou les sels de sodium et de calcium de saccharine, le cyclamate de sodium, le saccharose, le fructose, le glucose, le sorbitol et leurs mélanges ; l'édulcorant préféré est le sucralose.

21. Comprimé à désintégration orale selon la revendication 20, dans lequel le sucralose est présent en une quantité de 0,01 à 5,0% en poids de la composition totale, de préférence de 0,05 à 2,0% en poids de la composition totale.

22. Comprimé à désintégration orale selon la revendication 13, dans lequel les agents aromatiques sont choisis parmi les arômes de fruits tels que l'orange, la banane, la fraise, la cerise, la cerise sauvage, le citron et d'autres arômes tels que la cardamome, l'anasone, la menthe, le menthol, la vanilline et éthylvanilline, et leurs mélanges, l'agent aromatique préféré est choisi parmi les arômes de fruits, tels que l'arôme d'orange.

23. Comprimé à désintégration orale selon la revendication 22, dans lequel l'agent aromatique est présent en une quantité de 0,05 à 5,0% en poids de la composition totale.
